# EUROPEAN PATENT APPLICATION

(11) **EP 2 823 811 A1**
(43) Date of publication of application: **14.01.2015**
(21) Application number: 13175685.0
(22) Date of filing: 09.07.2013
(51) Int. Cl.: A61K 9/51

(54) **Targeted active release system comprising solid lipid nano-particles**

(71) Applicant: OTC GmbH, 46047 Oberhausen (DE)
(72) Inventor: Dahms, Gerd, 47138 Duisburg (DE)
(74) Representative: Michalski Hüttermann & Partner Patentanwälte mbB

(57) **Abstract**

The present invention relates to lamellar encapsulated nano-particles comprising at least one liquid crystalline emulsifier membrane around an inner core, wherein the inner core is solid below 30°C and comprises at least one substance selected from the group consisting of lipids, waxes and/or polymeric substances, wherein the particle size is ≥ 100 nm and ≤ 800 nm and the average packing parameter of the emulsifier in the liquid crystalline membrane is ≥0.3 and ≤ 0.9.

## Description

### FIELD OF THE INVENTION

The present invention relates to lamellar encapsulated nano-particles comprising at least one liquid crystalline emulsifier membrane around an inner core, wherein the inner core is solid below 30°C and comprises at least one substance selected from the group consisting of lipids, waxes and/or polymeric substances, wherein the particle size is ≥ 100 nm and ≤ 800 nm and the average packing parameter of the emulsifier in the liquid crystalline membrane is ≥ 0.3 and ≤ 0.9.

### BACKGROUND OF THE INVENTION

The challenges of providing a suitable amount of helpful substances in a suitable active state at the right spot are well known to formulation chemists in all industrial areas. This statement is valid for a lot of different applications, where better effects or higher efficacy is desired. Usually it is not the problem to find substances with higher impact, but, nevertheless, also usually the side conditions like stability of the substance in the chosen environment and the accessibility of the targeted spot of action may provide the real problem.

In order to overcome those obstacles a lot of different technical solutions have been proposed, among which several has been especially designed for the cosmetic or pharmaceutical industry. But, the fact that such solutions has evolved from these sectors doesn't necessarily mean that other sectors, like e.g. industrial cleaning, crop science or the dye industry cannot participate from such solutions.

One technical remedy can be seen in the provision of stabilized ingredients, which are less prone to chemical degradation. This can be achieved either by a direct covalent modification of the molecule or by association of the molecule to other compounds. One example is for instance given in US2010331260 A1, which relates to a stabilized vitamin C derivative with a peptide molecule linked to vitamin C or a pharmaceutically acceptable salt thereof, a method of preparing the same, and a composition containing the same.

A more complex solution for the stabilization of vitamin c is provided in US2008319060 A1. The composition according to this invention includes cationic and anionic material as a primary stabilizing agent of vitamin C; and a caffeic acid derivative as a secondary stabilizing agent of vitamin C. The caffeic acid derivative is water-soluble and, preferably, a new caffiec acid derivative is used. According to this document, the cationic material and the anionic material generate an electrical double layer to stabilize the vitamin C primarily; water-soluble caffeic acid derivative stabilizes the vitamin C secondarily. Accordingly, the vitamin C is stabilized double so that the vitamin C is protected from being oxidized by air, heat and moisture.

A different strategy for retinol stabilization and delivery in topical application is for instance described in EP2583665 A2. In this document a method for stabilizing retinol (Vitamin A), an unstable fat-soluble material, and the use of the same in cosmetics is described. The document provides an anti-inflammatory and skin wrinkle reducing cosmetic composition containing retinol stabilized by nano-emulsification, wherein a retinol polymer nanocapsule formed by capturing retinol with porous polymer particles is nano-emulsified by a mung bean MCT (medium chain triglyceride) extract and lecithin.

Especially liposome based protection systems are in the focus of the pharmaceutical industry. EP1924247 A2 for instance discloses a method of liposome-based therapy for a mammalian subject. The method uses liposomes and/or liposomes with outer surfaces that contain an affinity moiety effective to bind specifically to a biological surface at which the therapy is aimed, and a hydrophilic polymer coating. The hydrophilic polymer coating is made up of polymer chains covalently linked to surface lipid components. After a desired liposome biodistribution is achieved, the affinity agent binds to the target surface and helps internalize the liposomes. Therefore, not only the stability but also the targeted release is addressed by such solution.

In particular US5885486 A describes special administration forms and delivery systems for drugs, vaccines and other biologically active agents. More specifically this document is related to the preparation of suspensions of colloidal solid lipid particles (SLPs) of predominantly anisometrical shape with the lipid matrix being in a stable polymorphic modification and of suspensions of micron and submicron particles of bioactive agents (PBAs); as well as to the use of such suspensions or the lyophilizates thereof as delivery systems primarily for the parenteral administration of preferably poorly water-soluble bioactive substances, particularly drugs, and to their use in cosmetic, food and agricultural products. SLPs and PBAs are prepared by an emulsification process, wherein: (1) A solid lipid or bioactive agent or a mixture of solid lipids or bioactive agents is melted. (2) Stabilizers are added either to the lipid or bioactive agent and to the aqueous phase or to the aqueous phase only depending on their physicochemical characteristics. Stabilizers may also be added or exchanged after homogenization. (3) Drugs or other bioactive substances to be incorporated into the SLPs may be melted together with the lipids if the physicochemical characteristics of the substance permit or may be dissolved, solubilized or dispersed in the lipid melt before homogenization. (4) The aqueous phase is heated to the temperature of the melt before mixing and may contain for example stabilizers, isotonicity agents, buffering substances, cryoprotectants and/or preservatives. (5) The molten lipid compounds and the bioactive agents are emulsified in an aqueous phase preferably by high-pressure homogenization. Nevertheless, usually such SLPs are prepared using a high pressure homogenization (HPH) step during processing. Such high pressure step can harm active ingredients, degrade polymer and is additionally able to generate free radicals (see for instance Landere et al., Gaulin Homogenization: a mechanistic study, Biotechnol. Prog. 16 (2000) 80-85). Therefore, such high pressure homogenization step during processing is disadvantageous in not in the sense of the invention.

Such solutions are usually effective in the chemical stabilization of the ingredient. Nevertheless, in most cases it cannot be excluded, that also the bioavailability profile might be changed due to the proposed complex formation or even the chemical modification. In addition, regularly often the targeted release is not addressed, resulting in a less effective delivery system.

### SUMMARY OF THE INVENTION

Therefore, it is the task of the present invention to provide a flexible and effective encapsulation system for sensitive ingredients, which is cost effective and can be used in a wide variety of different application fields.

### DETAILED DESCRIPTION OF THE PREFERRED EMBODIMENTS

This object is achieved according to the invention by the provision of a lamellar encapsulated nano-particle comprising at least one liquid crystalline emulsifier membrane around an inner core, wherein the inner core is solid below 30°C and comprises at least one substance selected from the group consisting of lipids, waxes and/or polymeric substances, **characterized in that** the particle size is ≥ 100 nm and ≤ 800 nm and the average packing parameter of the emulsifiers in the liquid crystalline membrane is ≥ 0.3 and ≤ 0.9. Surprisingly it has been found that such lamellar encapsulated nano-particles are compatible with a wide range of different chemical surroundings, like aqueous solutions, oils, silicones or the known variety of emulsion-systems, for instance w/o- (water in oil), o/w- (oil in water) or triplet emulsions like w/o/w or o/w/o-type. The particles can be easily incorporated in such systems by a simple low shear mixing process as for instance provided by an anchor or blade stirrer and the particles remain stable in such systems for a prolonged period of time. Therefore, the combination of the lamellar encapsulated nano-particles with any kind of liquid is easy and can be achieved without high investment costs. Therefore it is easy to generate diluted solutions, which might be suitably used as product. The lamellar encapsulated nano-particles can reversibly be diluted and re-concentrated to nearly any concentration regime with polar or apolar fluids and at low liquid contents the particles form an isotropic (optically clear or slightly opaque) cubic phase. Without being bound by the theory such cubic phase may especially be generated by the alignment of the encapsulated particles on surfaces either in the form of a mono-layer or as a stacked cubic dense packing. The overall composition is compatible with a wide range of surfaces and such lamellar encapsulated nano-particles can especially be applied to surfaces of the human body like skin or hair. Here especially a dense surface film may be helpful in order to mechanically protect the skin surface or in order to reduce the trans-epidermal water loss (TEWL). Due to their solid inner core, the particles remain unchanged in size and the size distribution of the particles remains very stable during storage.

The nano-particle is encapsulated, i.e. surrounded, by an emulsifier mono-layer directly on the surface of the lipid inner core and additionally surrounded by a least one lamella, which consists of a bi-layer of emulsifier molecules. Without being bound by the theory the lamellae can be formed by a bending of a worm-like micelle around the lipid inner core in the course of particle processing. Each lamella is formed by two emulsifier layers, which are in contact to each other by their hydrophobic tails, facing inwards into the lamella. The hydrophilic emulsifier head groups are facing outwards, thus forming two hydrophilic bend surfaces. The inner lipid core can be surrounded by at least 1, up to 50, preferably up to 25 and most preferably 1 up to 10 lamellae.

The emulsifier membrane is in a liquid crystalline state, i.e. the properties of the emulsifier molecules are in between those of conventional liquids and those of a solid crystal. Due to van-der-Waals interactions the single emulsifier molecule is connected to the surrounding emulsifier molecules, thus forming a phase behavior of the membrane which is in between a liquid and a crystal. Such behavior influences the solubility of other substances in the membrane and physical parameter like the melting or the viscosity. Furthermore, the fact that the particle is surrounded by the emulsifier membrane enables the particles to be self-emulsifying in an aqueous environment.

The inner core of the lamellar encapsulated nano-particle is solid. Solid in the sense of the invention means, that the inner core exhibits a at room temperature (20°C) a viscosity higher than 10⁴ mPa s and upon heating an endothermic peak is visible in a DSC-thermogramm, indicationg the phase transition. The core of the particle is surrounded at all sides by emulsifier molecules, thus preventing a direct contact of the lipid core to a large amount of solvent molecules.

The inner core of the lamellar encapsulated nano-particles may be formed by lipids, waxes and/or polymeric substances. These substances include lipids and lipid-like structures. Examples of suitable lipids are the di- and triglycerides of saturated straight-chain fatty acids having 12 to 30 carbon atoms, such as lauric acid, myristic acid, palmitic acid, stearic acid, arachidic acid, behenic acid, lignoceric acid, cerotic acid and melesinic acid, and their esters with other saturated fatty alcohols having 4 to 22, preferably 12 to 22 carbon atoms such as lauryl alcohol, myristyl alcohol, cetyl alcohol, stearyl alcohol, arachidyl alcohol, behenyl alcohol, saturated wax alcohols having 24 to 30 carbon atoms such as lignoceryl alcohol, cetyl alcohol, cetearyl alcohol and myristyl alcohol. Preference is given to di- and triglycerides, fatty alcohols, their esters or ethers, waxes, lipid peptides or mixtures thereof. Use is made in particular of synthetic di- and triglycerides as individual substances or in the form of a mixture, such as in the form of a hard fat, for example. Examples of glyceryl tri-fatty acid esters are glyceryl trilaurate, glyceryl trimyristate, glyceryl tripalmitate, glyceryl tri-stearate or glyceryl tribehenate. Waxes which can be used in accordance with the invention are natural waxes, such as plant waxes, animal waxes, mineral waxes and petrochemical waxes, chemically modified waxes, such as hard waxes, and synthetic waxes. For a listing of suitable waxes reference may be made to Römpp Chemielexikon, 9th edition, entry "Waxes". Examples of suitable waxes are beeswax, carnauba wax, candelilla wax, paraffin waxes, isoparaffin waxes, and rice wax. Further examples of suitable waxes are cetyl palmitate and cera alba (bleached wax, DAB [German Pharmacopeia] 9). Suitable esters derive further, for example, from branched-chain fatty acids and fatty alcohols, glycerol, sorbitan, propylene glycol, methylglycoside, citric acid, tartaric acid, and mellitic acid. It is further possible to use ceramides, phytosphingosides, cholesterol, and phytosterols.

In addition, it is further possibility is to use polymers such as silicone waxes and PVP derivatives for buiding the inner core. These are, for example, alkyl-substituted PVP derivatives, examples being tricontanyl-PVP, PVP-hexadecene copolymer, and PVP/eicosene copolymer. They can be used, for example, alone or as admixtures to the lipids as inner core materials. It is also possible to use solid urethane derivatives, such as are sold, for example, by ALZO International Inc. These include, for example, fatty alcohol (branched) dimer/IPDI, fatty alcohol (linear) dimer/IPDI, ethoxylated fatty alcohol (branched) dmer/IPDI, ethoxylated fatty alcohol (linear) dimer/IPDI, dimethiconol/IPDI copolymers, triglyceride ester (hydrogenated)/IPDI copolymers, ethoxylated triglyceride ester (hydrogenated)/IPDI copolymers, aminated ethoxylated and non-ethoxylated triglyceride ester/IPDI copolymers.

The particle size of the lamellar encapsulated nano-particles is ≥ 100 nm and ≤ 800 nm. This means, that the longest distance between two points in the particle, i.e. the inner core and the surrounding lamellae, is within said range. If the particle exhibits a spherical shape, this size can be interpreted as the diameter of the particle. Although the sizes of the particles can be very uniform, i.e. that all particles exhibit the same size within an uncertainty of ± 10 nm, also a range of sizes, i.e. a size distribution of the lamellar encapsulated nano-particles is within the scope of the invention. In that case at least 95 weight % of the particles may exhibit sizes in the above given range. The sizes or the size distribution of the lamellar encapsulated nano-particles can be determined in aqueous solution using laser light scattering techniques (at 20 °C, 2 weight% lamellar encapsulated nano-particles in de-mineralized water at pH 7.0). In order to calculate the size distribution a mono-modal mathematical model of particles with spherical symmetry can be used. If such mathematical assumptions are not valid the size and the size distribution can otherwise be assessed using electron microscopy on freeze-fractured samples, followed by optical evaluation.

According to the invention the average packing parameter of the emulsifier in the liquid crystalline membrane is ≥ 0.3 and ≤ 0.9. The packing parameter for the emulsifier is determined according to Israelachvili and is given by the ratio of the emulsifier volume (V) and the product of the tail length and the head-group surface area (a₀*l_{c}), packing parameter = V/ (a₀*l_{c}). It has surprisingly been found that such range of emulsifier packing parameter is able to ease the lamellar phase formation and results in fast and stable lamellae formation. This result is achievable without the need to introduce high shear mixing within the single mixing areas. This reduces production costs and processing times. Furthermore, such packing parameter may simplify a cubic phase formation on biological surfaces, like the skin. Furthermore, it is unlikely that such emulsifiers with a packing parameter in that range are able to be effectively incorporated into the skin lipid membrane, hence these emulsifier are less prone to cause side effects or irritations on biological surfaces. Higher packing parameter will ease the incorporation in biological membranes and therefore are less preferred. Smaller packing parameter may lead to only not sufficiently stable membranes. Examples of the calculation of the packing parameter of different emulsifier can be found in Israelachvili, J. N.; Mitchell, D. J.; Ninhem, B. W. J. Chem. Soc.,Faraday Trans 2 1976, 72, 1525. One example for an emulsifier suitable to be used within the invention, i.e. exhibiting a packaging parameter in that range, is sodium dodecyl sulfate. The following molecular characteristics are tabulated: lc = 1.67 nm; a₀ = 0.57 nm²; V = 1.0.3502 nm³ resulting in a packing parameter of 0.37.

As emulsifiers which form lyotropic lamellar structures it is possible to use natural or synthetic products. The use of surfactant mixtures is a further possibility. Examples of suitable emulsifiers are the physiological bile salts such as sodium cholate, sodium dehydrocholate, sodium deoxycholate, sodium glycocholate, and sodium taurocholate. Animal and plant phospholipids such as lecithins together with their hydrogenated forms, and also polypeptides such as gelatin, with their modified forms, may also be used.

Suitable synthetic surface-active substances are the salts of sulfosuccinic esters, polyoxyethylene acid betaine esters, acid betaine esters and sorbitan ethers, polyoxyethylene fatty alcohol ethers, polyoxyethylenestearic esters, and corresponding mixture condensates of polyoxyethylene-methpolyoxypropylene ethers, ethoxylated saturated glycerides, partial fatty acid glycerides and polyglycides. Examples of suitable surfactants are Biobase(R) EP and Ceralution(R) H.

Examples of suitable anionic emulsifiers are Salts of alkyl sulfates, alkylether sulfates, mono-, di or tri phosphate esters, alkyl sulfosuccinate, alkyl lactylates such as sodium lauroyl lactylate or sodium stearoyl lactylate, amino acid-derived anionic surfactants such as sodium cocoyl glycinate or sodium cocoyl hydrolyzed wheat protein, acyl isethionates, acyl taurates, alkyl ether carboxylates.

Examples of suitable cationic emulsifiers are behentrimonium chloride, cetrimonium chloride, behenoyl pg-trimonium chloride, lauroyl pg-trimonium chloride, distearyldimonium chloride, distearoylethyl dimonium chloride, palmitamidopropyltrimonium chloride.

Examples of suitable amphoteric emulsifiers are amphoacetate, betaines, amidopropyl betaines, alkyl aminoxides, coconut imidazoline dicarboxymethylated, alkyl hydroxysultaine.

Examples of suitable nonionic emulsifiers alkyl polyglucoside, polyglcerinesters, sorbitanesters, sorbitolesters, methylglucosidesters, glucoseesters ethoxylated fatty alcohols, ethoxylated fatty acids, ethoxylated fatty amines, ethoxylated fatty amides, ethoxylated mono, di, triglycerides, ethoxylated sorbitanester, ethoxylated sorbitolesters such as peg sorbitol peroleate, ethoxylated polyglycerinesters, ethoxylated methylglucosideesters, EO-PO-copolymers and fatty acid amides, fatty acid amides reacted with ethanolamines such as cocamide mea, cocamide dea, glucamide.

In a preferred characteristic of the inventive particle the particle shape may be selected from the group consisting of cubic, oblong, disc like or ellipsoid. Especially the inventive lamellar encapsulated nano-particles may form effective layers on surfaces if the shape of the particles is not sphere like. As a consequence such particles may show a better alignment on the surface and hence may exhibit an enhanced protective layer. Especially in the case of dermatological application such particle geometry might lead to a stable layer, which may reduce the TEWL of the skin surface. In addition, such geometry may lead to a higher contact area between the particle and the surface, which may ease a preferred breakdown of the emulsifier membrane and a better contact of the inner core. The geometry of the particles can easily be accessed by freeze-fracture electron-microscopy or small-angle X-ray scattering techniques.

In an additional embodiment according to the invention the particle size distribution of the lamellar encapsulated nano-particle may comprise a full width at half maximum of ≥ 0 nm and ≤ 100 nm. Especially lamellar encapsulated nano-particles, which exhibit one size or a very narrow size distribution may be very well suited in order to build a dense layer on surfaces. The narrow size distribution may reduce the possibility of packing defects and may therefore result in an effective protective layer. The full width at half maximum can be determined mathematically based on the sized distribution obtained by MALLS (multi angle laser light scattering) as known to the skilled in the art. In a first approach the size distribution can be calculated according to a mono-modal distribution of sphere-like particles.

In another preferred aspect of the invention the solid inner core of the particle may be essentially free of emulsifiers. Essentially free in the sense of the invention means that the emulsifier concentration in the inner lipid core is less than 5 weight%. Such low emulsifier concentration is especially preferred, because it may result in a sharp melting profile of the inventive particles. In addition, if further active compounds (in the following also referred to as actives) may be present in the inner lipid core low emulsifier contents in the core may lead to a better controlled release of the active. Such behavior can especially not be expected, if the emulsifier is also present at higher amounts in the inner lipid core. Such situation may lead to a further encapsulation of the active, which may consequently lead to a reduced penetration of active out of the inner lipid core. The emulsifier concentration in the inner lipid core can be determined chemically after thoroughly washing of the dried lamellar encapsulated nano-particles with demineralized water followed by a quantitative HPLC.

In an additional characteristic of the inventive particle the melting temperature of the inner core may be ≥ 35°C and ≤ 55°C. Such range of melting temperatures of the inner core of the lamellar encapsulated nano-particles is especially preferred, because this range assures a high stability of the geometry of the particle even at elevated temperatures and therefore a sufficient stability of the overall system. Lower melting temperatures may be unfavorable, because the diluted particles may deform upon storage or diluted in solution and may therefore yield just an insufficient dense film formation on surfaces. Higher melting temperatures are less preferred, because the fluidity of the inner lipid core might remain too high, resulting in only a partial interaction of the inner core with the surface.

Furthermore, one additional preferred embodiment of the invention includes a particle, wherein the particle comprises an additional active. The additional active can be either hydrophilic or hydrophobic in nature and can be incorporated either in the inner lipid core, in the particle lamellae or in between the different lamellae. Such encapsulation may lead to an enhanced stability of the active due to the controlled, non oxidative environment, which may also exhibit a low water activity. The active can be further introduced in order to dye or to scent particle or to add further functionalities to the lamellar encapsulated nano-particle. Such further functionalities can for instance be directed to a skin care or pharmaceutical treatment using pharmaceutical or skincare actives. I.e. Analgesics/anti-inflammatories, such as morphine, codeine, piritramide, fentanyl and fentanyl derivatives, levomethadone, tramadol, diclofenac, ibuprofen, indometacin, naproxen, piroxicam, penicillamine; antiallergics, such as pheniramine, dimetindene, terfenadine, astemizole, loratadine, doxylamine, meclozine, bamipine, clemastine; antibiotics/chemotherapeutics, such as polypeptide antibiotics such as colistin, polymyxin B, teicoplanin, vancomycin; antimalarials such as quinine, halofantrin, mefloquine, chloroquine, virostatics such as ganciclovir, foscarnet, zidovudine, aciclovir and others such as dapsone, fosfomycin, fusafungine, trimetoprim; antiepileptics, such as phenyloin, mesuximide, ethosuximide, primidone, phenobarbital, valproic acid, carbamazepine, clonazepam; antimycotics, such as internals: nystatin, natamycin, amphotericin B, flucytosine, miconazole, fluconazole, itraconazole; and externals: clotrimazole, econazole, tioconazole, fenticonazole, bifonazole, oxiconazole, ketoconazole, isoconazole, tolnaftate; corticoids (internals), such as aldosterone, fludrocortisone, betamethasone, dexamethasone, triamcinolone, fluocortolone, hydroxycortisone, prednisolone, prednylidene, cloprednol, methylprednisolone; dermatologic agents, such as antibiotics: tetracycline, erythromycin, neomycin, gentamycin, clindamycin, framycetin, tyrothricin, chlortetracycline, mupirocin, fusidic acid; virostatics as above, and also: podophyllotoxin, vidarabine, tromantadine; corticoids as above, and also: amcinonide, fluprednidene, aclometasone, clobetasol, diflorasone, halcinonid, fluocinolone, clocortolone, flumethasone, difluocortolone, fludroxycortide, halometasone, desoximetasone. fluocinolide, fluocortin butyl, fluprednidene, prednicarbate, desonide; diagnostic agents, such as radioactive isotopes such as Te99m, In111 or I131, covalently bonded to lipids or lipoids or other molecules or in complexes, highly substituted iodine-containing compounds such as, for example, lipids; hemostyptics, such as blood coagulation factors VIII, IX; hypnotics, sedatives, such as cyclobarbital, pentobarbital, phenobarbital, methaqualone, benzodiazepines (flurazepam, midazolam, netrazepam, lormetazepam, flunitrazepam, trazolam, brotizolam, temazepam, loprazolam); hypophyseal hormones, hypothalamus hormones, regulatory peptides and their inhibitors, such as corticotrophin, tetracosactide, chorionic gonadotropin, urofollitropin, urogonadotropin, somatropin, metergoline, bromocriptine, terlipressin, desmopressin, oxytocin, argipressin, ornipressin, leuprorelin, triptorelin, gonadorelin, buserelin, nafarelin, goselerin, somatostatin; immunotherapeutics and cytokines, such as dimepranol 4-acetamidobenzoate, thymopentin, [alpha]-interferon, [beta]-interferon, filgrastim, interleukins, azathioprine, ciclosporin; local anesthetics, such as internals: butanilicaine, mepivacaine, bupivacaine, etidocaine, lidocaine, articaine, prilocalne; and externals: propitocaine, oxybuprocaine, etracaine, benzocaine; antimigraine agents, such as proxibarbal, lisuride, methysergide, dihydroergotamine, clonidine, ergotamine, pizotifen; narcotics, such as methohexital, propofol, etomidate, ketamine, alfentanil, thiopental, droperidol, fentanyl; parathyroid hormones, calcium metabolism regulators, such as dihydrotachysterol, calcitonin, clodronic acid, etidronic acid; ophthalmic agents, such as atropine, cyclodrine, cyclopentolate, homatropine, tropicamide, scopolamine, pholedrine, edoxudine, idoxuridine, tromantadine, aciclovir, acetazolamide, diclofenamid, carteolol, timolol, metipranolol, betaxolol, pindolol, befunolol, bupranolol, levobunolol, carbachol, pilocarpine, clonidine, neostigmine; psychopharmaceuticals, such as benzodiazepines (lorazepam, diazepam), clomethiazole; thyroid gland therapeutic agents, such as 1-thyroxine, carbimazole, thiamazole, propylthiouracil; sera, immunoglobulins, vaccines, such as immunoglobulins generally and specifically such as hepatitis types, German measles, cytomegalovirus, rabies; TBE, varicella zoster, tetanus, rhesus factors, immune sera such as botulism antitoxin, diphtheria, gas gangrene, snake poison, scorpion venom, vaccines, such as influenza, tuberculosis, cholera, diphtheria, hepatitis types, TBE, German measles, Haemophilus influenzae, measles, Neisseria, mumps, poliomyelitis, tetanus, rabies, typhus; sex hormones and their inhibitors, such as anabolics, androgens, antiandrogens, gestagens, estrogens, antiestrogens (tamoxifen etc.); cytostatics and metastase inhibitors, such as alkylating agents such as nimustine, melphalan, carmustine, lomustine, cyclophosphamide, ifosfamide, trofosfamide, chlorambucil, busulfan, treosulfan, prednimustine, thiotepa, antimetabolites such as cytarabine, fluorouracil, methotrexate, mercaptopurine, tioguanine, alkaloids such as vinblastin, vincristin, vindesin; antibiotics such as aclarubicin, bleomycin, dactinomycin, daunorubicin, epirubicin, idarubicin, mitomycin and plicamycin, complexes of transition group elements (for example Ti, Zr, V, Nb, Ta, Mo, W, Pt) such as carboplatin, cisplatin, and metallocene compounds such as titanocene dichloride, amsacrine, dacarbazine, estramustine, etoposide, hydroxycarbamid, mitoxantrone, procarbazine and temiposide, alkylamido phospholipids (described in J. M. Zeidler, F. Emling, W. Zimmermann and H. J. Roth, Archiv der Pharmazie, 324 (1991), 687), and ether lipids such as hexadecylphosphocholine, ilmofosine and analogs, described in R. Zeisig, D. Arndt and H. Brachwitz, Pharmazie 45 (1990), 809 to 818.

Examples of further suitable active compounds include diclofenac, ibuprofen, acetylsalicylic acid, salicylic acid, erythromycin, ketoprofen, cortisone, and glucocorticoids. Additionally suitable are active cosmetic compounds, which in particular are sensitive to oxidation or hydrolysis, such as polyphenols, for example. Mention may be made here of catechins (such as epicatechin, epicatechin 3-gallate, epigallocatechin, epigallocatechin 3-gallate), flavonoids (such as luteolin, apigenin, rutin, quercitin, fisetin, kaempherol, rhamnetin), isoflavones (such as genistein, daidzein, glycitein, prunetin), coumarins (such as daphnetin, umbelliferone), emodin, resveratrol, and oregonin.

Suitable vitamins include retinol, tocopherol, ascorbic acid, riboflavin, and pyridoxine.

Suitability is possessed, furthermore, by whole extracts from plants that include above molecules or classes of molecule.

Additionally, the active may also provide sunscreen activity. Suitable sunscreen agents (UV filters) are, for example, compounds based on benzophenone, diphenyl cyanoacrylate or p-aminobenzoic acid. Specific examples are (INCI or CTFA names) Benzophenone-3, Benzophenone-4, Benzophenone-2, Benzophenone-6, Benzophenone-9, Benzophenone-1, Benzophenone-11, Etocrylene, Octocrylene, PEG-25 PABA, Phenylbenzimidazole Sulfonic Acid, Ethylhexyl Methoxycinnamate, Ethylhexyl Dimethyl PABA, 4-Methylbenzylidene Camphor, Butyl Methoxy-dibenzoylmethane, Ethylhexyl Salicylate, Homosalate, and Methylene-Bis-Benzotriazolyl Tetramethylbutylphenol (2,2'-methylene-bis {6-(2H-benzotriazol-2-yl)-4-(1,1,3,3-tetramethylbutyl)phenol}, 2-hydroxy-4-methoxy-benzophenone-5-sulfonic acid, and 2,4,6-trianilino-p-(carbo-2'-ethylhexyl-1'-oxy)-1,3,5-triazine.

Further organic sunscreen agents are octyltriazones, avobenzones, octyl methoxycinnamates, octyl salicylates, benzotriazoles, and triazines.

Such inventive lamellar encapsulated nano-particles are especially suited to incorporate oxygen or hydrolysis sensitive actives. Furthermore, the inventive particles may lead to a controlled delivery of the encapsulated active into the skin. Without being bound by the theory the action of the lamellar encapsulated nano-particles on the dermis can be twofold. On the one hand the particles may form a dense film on the surface after the outer phase has partially evaporated. Such film can at best be described as a cubic dense packing of the single lamellar encapsulated nano-particles. Due to the film formation the TEWL is reduced, leading to a gentle disturbance of the protective lipid bi-layer of the skin, thus enhancing penetration. In addition, due to an uptake of skin lipids, which are present on the skin surface (for instance squalane), the outer lamellae structure may be disturbed, which in turn may lead to a direct contact of the lipid core with the skin. Consequently, also actives incorporated within the inner core are able to penetrate into the skin. The trigger of this process might be seen by a fluidization of the lamellar structure caused by the uptake of the skin lipids. Therefore, the release of the actives in the inner core is triggered. In such way different penetration profiles can be designed as a function of the active distribution between the lamellae and the inner lipid core. Furthermore, also the melting temperature of the inner lipid core can be used to further modulate the penetration profile of the active.

Furthermore, a particle is within the scope of the invention, wherein the active is incorporated in the inner lipid core in an amount of ≥ 5 weight% and ≤ 95 weight % with respect to the total active amount. Such distribution of the active compound between the inner core and the outer emulsifier lamellae might lead to an even release profile from the lamellar encapsulated nano-particles to surfaces. Such release profile might in consequence lead to an even penetration profile of the active. Taking for instance the penetration of actives into the skin surfaces into account such active distribution might first yield a rapid penetration of the actives which are located at the outer layers of the particle. After that the penetration is triggered from the more inner layers and in the last step the actives are released which are present in the inner lipid core. Thus an initial burst of the active is prevented and a prolonged active release is established. Higher active amounts in the inner core may be unfavorable in certain applications, because the release of the active at shorter timescale might be insufficient. Lower active amounts in the inner core might be unfavorable in certain applications, because in that case the long term release might be insufficient in order to achieve a significant effect. Preferably the active may be incorporated in the inner lipid core in an amount of ≥ 20 weight% and ≤ 90 weight % and more preferred in an amount of ≥ 40 weight% and ≤ 85 weight %. The concentration determination of the active in the inner lipid core and the lamellae can for instance be performed using quantitative HPLC after separation of the lipid inner core and the emulsifier lamellae.

Additionally the inventive particle may comprise an active, wherein the active is a hydrophilic active. It is especially suitable to incorporate hydrophilic actives in the inventive lamellar encapsulated nano-particles. Such actives may either be incorporated into the membrane or the inner part of the particle. The hydrophilic actives might for instance be dispersed at higher temperatures in the liquid lipid or wax and might be trapped within the inner core upon cooling. It is also possible to incorporate the hydrophilic active in the membrane part by adding the active in an aqueous phase or by mixing the active and the emulsifier. Due to the physical trapping of the hydrophilic active in the inner core, the release is triggered by a fluidization of the inner core. This for instance in skincare applications by the uptake of skin lipids which can be found on the outer skin surface. Due to the hydrophilic-hydrophobic mismatch of the active and the inner core the release and therefore also the penetration of the active is enhanced. This may lead to a faster and more effective penetration of the active from the inner core. Hydrophilic actives in the sense of the invention are actives which comprise a solubility in water at 20°C larger than 20 g/l.

Within a further object of the inventive particle an active may be added in a concentration of ≥ 0.5 weight% and ≤ 80 weight% with respect to the total particle weight. Efficacious treatment may be achievable by using said active concentration range. Lower concentrations are less preferred, because the effect of the active might be too low. Higher active concentrations might be unfavorable, because such high concentration might lead to instability of the inventive lamellar encapsulated nano-particle.

In another aspect of the invention the liquid crystalline membrane of the particle comprises at least two chemically different emulsifiers and the average C-chain length of both emulsifiers differs at least by 2 carbon-atoms. Such emulsifier composition of the liquid crystalline membrane might ease the triggered release of actives from the membrane structures or the inner lipid core itself. Without being bound by the theory the use of two different emulsifiers disturbs the packing of the membrane and thus might ease the uptake of other substances into the membrane, like for instance skin lipids present on the surface of the skin. Such composition is preferred in contrast to situations where only one emulsifier is present in the membrane. The difference in C-chain length between the different emulsifiers can be deduced from the difference in the average carbon chain length of the emulsifier. Therefore, where applicable the skilled in the art is able to obtain the special carbon chain length distribution of the different emulsifier and is able to calculate the average chain length. Both emulsifiers should differ with respect to their average carbon chain length.

In an additional embodiment of the inventive particle the liquid crystalline membrane of the particle comprises at least a C14-C22 emulsifier. Such carbon chain length of at least on emulsifier is preferred because such emulsifier is able to exhibit a suitable dense packing of the membrane during the storage time, thus protecting the inner lipid core. In addition such emulsifiers might also easily interact by the uptake of other lipid substances, like for instance skin surface lipids and consequently a triggered breakdown of the membrane might be achieved. Furthermore, such long tail emulsifiers are very suitable to establish the right emulsifier chain volume. Taking this chain volume into account a lot of different head groups are possible, without yielding a not preferred packing parameter. The C-chain length of the emulsifier may also exhibit a C 14-C20 and preferably a C 14- C18 carbon chain length.

Additionally, the inventive particle may comprise emulsifier, wherein the highest packing parameter of any emulsifier in the liquid crystalline membrane is less or equal 0.7. Surprisingly it has been found that the interaction of emulsifiers comprising said range of packing parameter are very suitable to form cubic dense phases in aqueous solutions on surfaces. Furthermore such emulsifiers are less prone to interact with skin lipids in the case of dermal application, which are responsible for in dermal barrier formation. Due to the packing parameter mismatch between such emulsifiers and for instance the skin ceramides it is less likely that the emulsifier membrane will uptake the ceramides from the skin lipid barrier and will induce a too intense barrier disruption. As a result the chosen emulsifier might contribute to a kin friendly composition of the overall formulation.

Furthermore, the use of a lamellar encapsulated nano-particle in painting-, varnish-, foodstuff , home care-, crop science-, cosmetic-, medicinal device- or pharmaceutical-applications is within the scope of the invention. Caused by the flexibility of the formulation system and the ability to protect sensitive substances against degradation in various environments such lamellar encapsulated nano-particles can duly be used in the above mentioned application areas. A further advantage can be achieved if surfaces are treated with the inventive lamellar encapsulated nano-particles, due to the formation of a protective cubic dense packing on the surface after the solvent has partially evaporated.

A use of the inventive particles is also within the scope of the invention, wherein the lamellar encapsulated nano-particle is dispersed into an aqueous-, oil- or emulsion-type-phase prior to application. The inventive lamellar encapsulated nano-particles can be processed and stored as highly concentrated solutions. This might ease the transport logistics. Due to the stability of the membrane protected particles these concentrated solutions do show no detectable tendency of particle coalescence. Such concentrated solution can easily be shipped and added to any kind of formulation either directly before the desired application or within a separate processing step prior to packaging. Therefore, it is also possible to establish a wider range of final formulations by the incorporation of differently loaded particles.

In addition, the lamellar encapsulated nano-particles can be used as a dermal delivery system for oxygen and hydrolysis unstable actives. Owing to the stability of the formulation system, and the triggered release on skin surfaces these nano-particles are especially suited to be used as a skin-friendly dermal delivery system. Such system provides enhanced protection of sensitive actives, which otherwise tends to degrade in unprotected environments. Oxygen unstable actives are substances comprising a loss of activity of higher than 10% within a one-month period at 25°C at ambient oxygen partial pressure. Hydrolysis unstable actives are substances comprising a loss of activity of higher than 10% within a one-month period at 25°C in water.

With respect to additional advantages and features of the previously described use of the particle it is explicitly referred to the disclosure of the inventive particle. In addition, also aspects and features of the inventive particle shall be deemed applicable and disclosed to the inventive use. Furthermore, all combinations of at least two features disclosed in the claims and/or in the description are within the scope of the invention unless otherwise explicitly indicated.

## Claims

1. Lamellar encapsulated nano-particle comprising at least one liquid crystalline emulsifier membrane around an inner core, wherein the inner core is solid below 30°C and comprises at least one substance selected from the group consisting of lipids, waxes and/or polymeric substances, **characterized in that** the particle size is ≥ 100 nm and ≤ 800 nm and the average packing parameter of the emulsifiers in the liquid crystalline membrane is ≥ 0.3 and ≤ 0.9.

2. Particle according to claim 1, wherein the shape of the particle is selected from the group consisting of cubic, oblong, disc like or ellipsoid.

3. Particle according to any of the preceding claims, wherein the particle size distribution of the lamellar encapsulated nano-particle comprises a full width at half maximum of ≥ 0 nm and ≤ 100 nm.

4. Particle according to any of the preceding claims, wherein the solid inner core is essentially free of emulsifiers.

5. Particle according to any of the preceding claims, wherein the melting temperature of the inner core is ≥ 35°C and ≤ 55°C.

6. Particle according to any of the preceding claims, wherein the particle comprises an additional active.

7. Particle according to claim 6, wherein the active is incorporated in the inner lipid core in an amount of ≥ 5 weight% and ≤ 95 weight % with respect to the total active amount.

8. Particle according to any of the claims 6 - 7, wherein the active is a hydrophilic active.

9. Particle according to any of the claims 6 - 8, wherein the active is added in a concentration of ≥ 0.5 weight% and ≤ 80 weight% with respect to the total particle weight.

10. Particle according to any of the preceding claims, wherein the liquid crystalline membrane comprises at least two chemically different emulsifiers and the average C-chain length of both emulsifiers differs at least by 2 carbon-atoms.

11. Particle according to any of the preceding claims, wherein the liquid crystalline membrane comprises at least a C 14-C22 emulsifier.

12. Particle according to any of the preceding claims, wherein the highest packing parameter of any emulsifier in the liquid crystalline membrane is less or equal 0.7.

13. Use of a lamellar encapsulated nano-particle according to any of the claims 1-12 in painting-, varnish-, foodstuff-, home care-, crop science-, cosmetic-, medicinal device-or pharmaceutical-applications.

14. Use according to claim 13, wherein the lamellar encapsulated nano-particle is dispersed into an aqueous-, oil- or emulsion-type-phase prior to application.

15. Use of a lamellar encapsulated nano-particle according to any of the claims 1-12 as a dermal delivery system for oxygen and hydrolysis unstable actives.
